# EUROPÄISCHE PATENTSCHRIFT

(11) **EP 1 185 683 B1**
(45) Veröffentlichungstag und Bekanntmachung des Hinweises auf die Patenterteilung: **05.11.2003**
(21) Anmeldenummer: 00925285.9
(22) Anmeldetag: 13.05.2000
(51) Int. Cl.: C12P 35/02, C12N 9/80

(54) **VERFAHREN ZUR VERZÖGERUNG DER DESAKTIVIERUNG VON GLUTARYLAMIDASE WÄHREND EINER ENZYMKATALYSE**
METHOD FOR DELAYING THE DEACTIVATION OF GLUTARYL AMIDASE DURING AN ENZYME CATALYSIS
PROCEDE POUR RALENTIR LA DESACTIVATION DE LA GLUTARYLAMIDASE PENDANT UNE CATALYSE ENZYMATIQUE

(30) Priorität: 28.05.1999 DE 19924632
(43) Veröffentlichungstag der Anmeldung: 13.03.2002
(73) Patentinhaber: Aventis Pharma Deutschland GmbH, 65929 Frankfurt am Main (DE)
(72) Erfinder: BONGS, Jürgen, D-65205 Wiesbaden (DE); MEIWES, Johannes, D-65510 Idstein (DE); KRUSE, Wolfgang, D-65719 Hofheim (DE); KOLLER, Klaus-Peter, D-65812 Bad Soden (DE)
(86) Internationale Anmeldenummer: EP0004337
(87) Internationale Veröffentlichungsnummer: WO00073490

(56) Entgegenhaltungen:
- ES-A- 2 093 555
- P. GOLINI ET AL.: "Immobilization of D-amino acid oxidase from different yeasts: characterization and application in the deamination of cephalosporin C" ENZYME AND MICROBIAL TECHNOLOGY, Bd. 17, Nr. 4, April 1995 (1995-04), Seiten 324-329, XP000925668 in der Anmeldung erwähnt
- EZIO BATTISTEL: "Purification and stability of Glutaryl-7-ACA acylase from Pseudomonas sp." APPLIED BIOCHEMISTRY AND BIOTECHNOLOGY, Bd. 69, Nr. 1, Januar 1998 (1998-01), Seiten 53-67, XP000925667

## Beschreibung

Die vorliegende Erfindung betrifft ein Verfahren zur Verzögerung der Desaktivierung von Glutarylamidase während einer Enzymkatalyse.

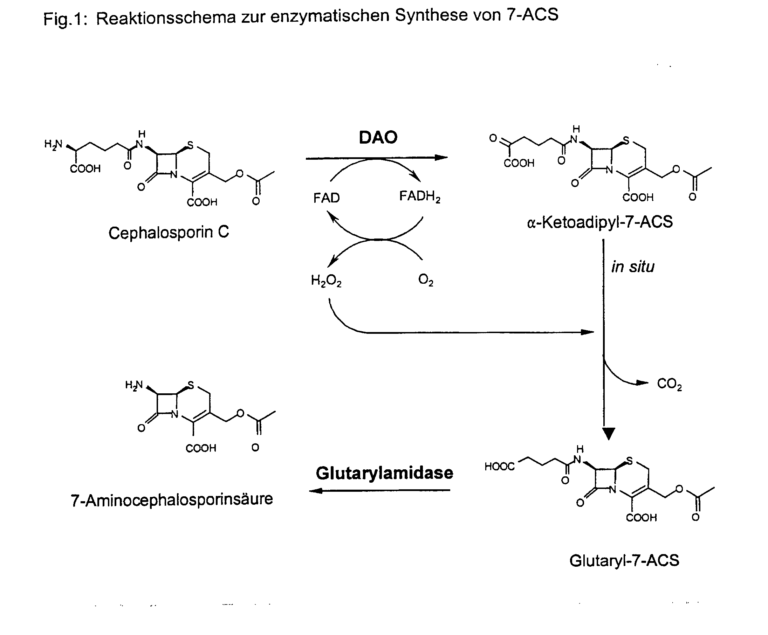

Die enzymatische Synthese der Antibiotikum-Vorstufe 7-Aminocephalosporinsäure (7-ACS) erfolgt - wie in Fig. 1 dargestellt - in zwei Reaktionsschritten. Zunächst wird Cephalosporin C unter Einwirkung von D-Aminosäureoxidase (DAO) zu α-Ketoadipyl-ACS oxidiert. Diese Verbindung wird im nachfolgenden Schritt durch durch die Glutarylamidase (GAE) zu 7-ACS hydrolisiert. 7-ACS ist von großem kommerziellem Interesse für die Produktion semisynthetischer Cephalosporinantibiotika.

Die Synthese erfolgt mit den in der Regel auf Trägern immobilisierten Enzymen D-Aminosäureoxidase bzw. Glutarylamidase. Diese Enzyme werden nach Beendigung der Reaktion von der Produktlösung abgetrennt und können erneut bei der nächsten Charge eingesetzt werden. Die mehrfache Benutzung der Enzyme führt allerdings zu deren Desaktivierung und entspricht einem Enzymverbrauch.

Aus der Literatur ist bekannt, daß strukturverändernde Oxidationen an Proteinen durch den Einsatz von Thiolreagenzien wie z.B. *2-Mercaptoethanol* (P.Golini et al. Enzyme and Microbial Technology, 17, 1995, 324-329; Int. J. Peptide Protein Res., 48, 1996, 532-538) unterdrückt werden können.
Beispielsweise lässt sich die D-Aminosäureoxidase (DAO) durch Thiole regenerieren. Das Flavoprotein DAO katalysiert die stereospezifische Deaminierung von D-Aminosäuren zu den entsprechenden α-Ketosäuren und Ammonium beispielsweise wie in Fig. 1 gezeigt die Umsetzung von Cephalosporin C zu α-Ketoadipyl-7-Aminocephalosporinsäure (α-Ketoadipyl-7-ACS). α-Ketoadipyl-7-ACS decarboxyliert in situ zu Glutaryl-7-ACS (P.Golini et al. Enzyme and Microbial Technology 17, 1995, 324 - 329). Bei der technische Anwendung erfolgt der Einsatz häufig nicht in gelöster Form sondern immobilisiert durch Bindung an Polymere wie z. B. aminoalkylierte Polymere oder oxiranaktivierte Polymere. Dadurch kann der Enzymkatalysator nach der Reaktion durch Filtration abgetrennt werden und steht für eine Wiederverwendung zur Verfügung. Der immobilisierte DAO-Katalysator unterliegt einer partiellen Inaktivierung. Wird der einmal verwendete Enzymkatalysator in einer weiteren Reaktion unter sonst gleichen Bedingungen wieder eingesetzt, verlängert sich die Reaktionszeit, die zur maximalen Umsetzung des Substrats nötig ist. Diese bei jeder Wiederverwendung auftretende Verlängerung der Reaktionszeit ist ein Maß für die Stabilität des Katalysators im Herstellprozess von z. B. Glutaryl-7-ACS. Das Ausmaß der Änderung der Reaktionszeit bis zum maximalen Substratumsatz ermittelt über mehrere Produktionszyklen wird als operationelle Stabilität bezeichnet. Die operationelle Stabilität der immobiliserten DAO lässt sich verbessern, wenn der Enzymkatalysator nach der Reaktion durch Filtration vom Reaktionsgemisch abgetrennt und mit einem Thiol, beispielsweise 2-Mercaptoethanol, versetzt wird. DAO enthält einige leicht oxidierbare Sulfhydrylgruppen, überwiegend als funktionelle Gruppen der Cystein-Aminosäuren des Proteins. Die Wirkung von 2-Mercaptoethanol beruht auf der reduzierenden Wirkung des Thiols gegenüber den oxidationsempfindlichen Sulfhydrylgruppen der Cysteine. Die Regeneration dieser oxidierten Sulfhydrylgruppen resultiert in einer deutlichen Verbesserung der operationellen Stabilität. Im besonderen Falle der DAO muss die Regenerierung nach Abtrennung des Enzyms vom Reaktionsgemisch erfolgen, da während der Enzymkatalyse H₂O₂ entsteht. Dieses würde als starkes Oxidationsmittel ein zugesetztes Thiol inaktivieren.

Die Zugabe von Thiol kann die Aktivität von Proteinen auch reduzieren. Auch dieser Effekt kann mit dem Vorhandensein von Cysteinresten erklärt werden. Ein Beispiel dafür ist die Aminoacylase. Die Aminoacylase ist ein dimeres Enzym mit einem Zn²⁺-Atom pro Untereinheit. Jede Untereinheit des Enzyms enthält 2 Cystein SH-Gruppen und 2 Disulfid-Bindungen. Die chemische Modifizierung der SH-Gruppen wie das Aufbrechen der Disulfid-Bindungen kann zu einer Inaktivierung des Enzyms führen Es konnte gezeigt werden, dass durch Zugabe von 2-Mercaptoethanol die Aktivität der Aminoacylase reduziert wird, wohingegen nach Entfernung des 2-Mercaptoethanol durch Dialyse oder Gelfiltration die ursprüngliche Enzymaktivität fast komplett wiederhergestellt werden kann (W. Kördel u. F. Schneider Biochem. Biophys. Acta 445, 1976, 446 - 457).

Da offensichtlich die Wirkung des Thiols durch die Oxidation bzw. Reduktion von Sulfhydrylgruppen von Cysteinresten vermittelt wird, sollte folglich die Zugabe von Thiolen zu Enzymen, von denen bekannt ist, dass sie keine Cysteinreste enthalten, zu keiner Änderung der Enzymaktivität führen.
Im Rahmen der mehrfachen Benutzung von GAE bei katalytischen Umsetzungen des Katalysators kommt es wie eingangs für die Synthese von 7-ACS beschrieben (vgl. Fig. 1) zu einer Desaktivierung des Enzyms, die einem Verbrauch entspricht. Die Stabilität des Katalysators korreliert mit wichtigen Produktionskosten des Prozesses wie dem Zeitbedarf, dem Abfallaufkommen und den Katalysatorkosten. Ein mit dem vorstehend für DAO beschriebenes vergleichbares, zur Stabilisierung von GAE bzw. zur Erhöhung deren operationellen Stabilität geeignetes Verfahren ist bisher nicht bekannt. Das Enzym GAE besteht aus 2 Peptidketten (Protein A und B). Die Interaktion der Ketten erfolgt über Wasserstoffbrückenbindungen, sowie hydrophile und hydrophobe Wechselwirkungen von Proteindomänen.

Applied Biochemistry and Biotechnology, 69(1), 1998, 53-67, Battistel offenbart ein Verfahren zur Reinigung von Glutaryl-7-ACA Acylase und deren Deaktivierung durch Guanidin.

Die Klonierung des Gens aus Pseudomonas sowie die Expression in E. coli wurde z.B in EP-P-0504798 (US5830743) sowie EP-A-0708180 (US5766881) beschrieben. Die Verwendung von Mikroorganismen oder Enzymen aus diesen zur Herstellung von 7-ACS findet sich in EP-A-0275901 (US4990444) sowie EP 0525861 B1 (US5332663). Ein Reinigungsverfahren für das Enzym ausgehend von einem überproduzierendem E. coli Stamm ist beschrieben in D. Bianchi et al., Enzyme and Microbiological Technology 20, 1997, 368 - 372.

Aufgabe der vorliegenden Erfindung ist es, ein Verfahren zur Verzögerung der Desaktivierung von Glutarylamidase während einer Enzymkatalyse bereitzustellen.

Wie der Aminosäureanalyse (Tabelle 1) zu entnehmen ist, fehlt der Glutarylamidase (GAE) die Aminosäure Cystein. Der Einsatz von Thiolreagenzien zur Stabilisierung sollte demnach bei diesem Biokatalysator keine Steigerung der operationellen Stabilität bewirken. Überraschenderweise konnte im Experiment das Gegenteil bewiesen werden. Der Zusatz unterschiedlicher thiolhaltiger Reagenzien (z.B. *2-Mercaptoethanol, Cystein)* führte bei der GAE je nach Konzentration zu einer drastischen Steigerung der operationellen Stabilität.

Demgemäß wird die Aufgabe der vorliegenden Erfindung gelöst durch ein Verfahren zur Verzögerung der Inaktivierung während einer Enzymkatalyse von Glutarylamidase, welches sich dadurch auszeichnet, dass das Enzym mit mindestens einem Thiol in Kontakt gebracht wird. Wahlweise kann das Enzym dabei in freier oder in an ein Trägermaterial gebundener Form vorliegen. Ein bevorzugtes Trägermaterial ist beispielsweise ein oxiran-aktiviertes Polyacrylat.

Unter Thiol oder Mercaptan wird eine chemische Verbindung wie 2-Mercaptoethanol, Glutathion oder die Aminosäure Cystein verstanden, deren Gemeinsamkeit darin besteht, dass sie eine Thiolgruppe (-SH) im Molekül enthalten. Trägergekoppelte GAE besteht aus dem Enzymkatalysator GAE, der beispielsweise an oxiran-aktivierte oder auch aminoalkylierte Polyacrylate (=Träger) gebunden vorliegt. Ein Verfahren zur Herstelllung trägergekoppelter GAE findet sich in D. Bianchi et al., Enzyme and Microbiological Technology 20, 1997, 368 - 372. Beispiele für Träger sind Eupergit®, Amberlite® XAD7 oder Duolite® A365 (alle Röhm und Haas).

Die vorliegende Erfindung betrifft ein Verfahren zur Verzögerung der Desaktivierung von Glutarylamidase während einer Enzymkatalyse, welches dadurch gekennzeichnet ist, dass das Enzym mit mindestens einem Thiol in Kontakt gebracht wird.
In einer bevorzugten Ausführungsform des Verfahrens ist die Glutarylamidase an einen polymeren Träger gekoppelt, bei welchem es sich insbesondere um ein oxironaktiviertes Polyacrylat handeln kann. Bevorzugt wird als Thiol 2-Mercaptoethanol verwendet, welches vorzugsweise im Konzentrationsbereich 1 bis 100 mM verwendet wird. In einer weiteren bevorzugten Ausführungsform des Verfahrens wird als Thiol Cystein eingesetzt, welches bevorzugt im Konzentrationsbereich 1 bis 100 mM zur Anwendung kommt.

Ein wie vorstehend bezeichnetes Thiol kann kontinuierlich oder diskontinuierlich verwendet werden. Bei der kontinuierlichen Verwendung ist das Thiol während der Reaktion zugegen, während bei der diskontinuierlichen Verwendung die Behandlung zwischen den Reaktionsschritten erfolgt. In einer bevorzugten Ausführungsform des Verfahrens der vorliegenden Erfindung wird als Substrat Glutaryl-7-Aminocephalosporinsäure verwendet. Die Glutaryl-7-cephalosporinsäure liegt bevorzugt im Bereich von 5-500 mM vor.

Das Verfahren der vorliegenden Erfindung eignet sich insbesondere zur Herstellung von 7-Aminocephalosporinsäure unter Verwendung von Glutarylamidase als Enzymkatalysator, wobei die Desaktivierung der Glutarylamidase wie vorstehend beschrieben erfolgt.

Die Erfindung betrifft weiterhin die Verwendung mindestens eines Thiols zur Verzögerung der Desaktivierung der Glutarylamidase gemäß einem Verfahren wie im vorhergehenden beschrieben.

### Beispiele:

Eine praktische Anwendungsmöglichkeit für eine Enzymkatalyse mittels Glutarylamidase besteht beispielsweise in der Herstellung von 7-Aminocephalosporinsäure (7-ACS) aus Glutaryl-7-aminocephalosporinsäure (vgl. Fig. 1). Die Enzymkatalyse wurde dabei wie folgt durchgeführt:
Das Substrat *Glutaryl-7-Aminocephalosporinsäue* (*G-7-ACS*) wurde in einer Konzentration von 40 mM bei 40 °C und pH 8,3 in einem Reaktionsvolumen von 120 ml mit der immobilisierten *GAE (aus Pseudomonas diminuta)* umgesetzt. Mit Hilfe eines automatisierten, peripheren Systems bestand die Möglichkeit, für den Prozess relevante Parameter (pH, Temperatur) on-line zu messen und durch eine geeignete Regelungsmimik zu kontrollieren. Der auf Grund der Reaktion auftretende pH-Shift ins Saure wurde durch Autotitration mit Lauge somit aufgefangen. Die Geschwindigkeit der Laugenzugabe korrelierte mit der Reaktionsgeschwindigkeit und konnte als Maß für den Umsetzungsgrad des Substrates (*G-7-ACS*) bewertet werden. Die PC-gestützte Datenverarbeitung erlaubte die on-line Bestimmung des Umsatzes und ergab das Abbruch-Kriterium der Reaktion. Der Abbruch der Reaktion wurde vorgenommen, als der maximalen Umsatz erreicht war. Das Erreichen des maximalen Umsatzes zeigte sich daran, dass keine Laugenzugabe mehr erforderlich war. Nach Erreichen des maximalen Umsatzes wurde der Katalysator von der Produktlösung über ein Filter getrennt und anschließend frische Substratlösung eingefüllt. Die Reaktionszeiten der einzelnen, aufeinanderfolgenden Umsetzungen (= Batches) konnten als Funktion der Batches aufgetragen werden. Über lineare Regression ergab sich eine Steigung der Geraden (= X-Koeffizient [min/Batch]), die als Maß für die Katalysatordesaktivierung bewertet werden konnte. Je kleiner dieser Wert, desto höher war die operationelle Stabilität des Katalysators.

### Beispiel 1: Reaktionszeit bis zu quantitativem Umsatz als Funktion der Batch-Nummer ohne bzw. mit Cystein

In mehreren Versuchsreihen wurde der Substratlösung *Cystein* als Thiolreagenz in unterschiedlichen Konzentrationen zugegeben, und die Reaktion wie beschrieben durchgeführt. Wie in der folgenden Tabelle zu sehen ist, nahm mit steigender *Cystein*-Konzentration die Geradensteigung und damit der X-Koeffizient ab Bei Annahme einer linearen Desaktivierung über die durchgeführten Umsetzungen hinaus ließ sich für jede Messreihe die Anzahl der möglichen Batches bis zur Verdopplung der Reaktionszeit ermitteln. Die Werte sind der nachfolgendenTabelle zu entnehmen. Man erkennt eine deutliche Steigerung der operationellen Stabilität, wenn der Reaktionsansatz zusätzlich Cystein enthält. Dies zeigt sich an der Zunahme der Anzahl durchgeführter einzelner Reaktionsansätze (=Batches), die durchgeführt werden können, bis die Reaktionszeit sich bis zur maximalen Substratumsetzung verdoppelt hat.

Die Reaktionsparameter wurden dabei wie folgt gewählt:

### Reaktionsparameter:

| | |
|---|---|
| V_{R} | = 120 ml |
| T | = 40 °C |
| pH | = 8,3 [Ammoniak 1,66 %] |
| [G-7-ACS] | = 40 mM [KPP 100 mM] |
| [Cystein] _{in Substrat} | = variabel |
| Menge (GAE) | = 500 U |

**Tabelle zu Beispiel 1:**

| Reaktionszeit bis zu quantitativem Umsatz als Funktion der Batch-Nummer *ohne* bzw. *mit* Cystein | | | | | |
|---|---|---|---|---|---|
| | *ohne* Cystein | *mit* Cystein [1 mM] | *mit* Cystein [6 mM] | *mit* Cystein [8mM] | *mit* Cystein [10 mM] |
| X-Koeffizient [min/Batch] | 0,414 | 0,131 | 0,077 | 0,046 | 0,016 |
| Anzahl der Batches bis zur Verdopplung der Reaktionszeit (bei linearer Desaktivierung) [-] | 36 | 114 | 195 | 326 | 938 |

Vergleich des X-Koeffizienten (d.i. die Zeitzunahme pro Batch) zum Erreichen quantitativen Umsatzes bzw. Anzahl der Batches bis zur Verdopplung der Reaktionszeit als Funktion der *Cystein*-Konzentration in der Substrat-Lösung

### Beispiel 2: Reaktionszeit bis zu quantitativem Umsatz als Funktion der Batch-Nummer ohne bzw. mit Mercaptoetahnol

*2-Mercaptoethanol* als Thiolreagenz führt zur Steigerung der operationellen Stabilität der GAE. Die folgende Tabelle zeigt den Vergleich der Versuchsläufe mit bzw. ohne *2-Mercaptoethanol*. Die Ergebnisse mit Thiol zeigen hinsichtlich der Stabilisierung der operationellen Stabilität einen vergleichbaren Verlauf wie mit Cystein.

Die gewählten Reaktionsparameter wurden folgendermaßen eingestellt:

### Reaktionsparameter:

| | |
|---|---|
| V_{R} | = 120 ml |
| T | = 40 °C |
| pH | = 8,3 [Ammoniak 1,66 %] |
| [G-7-ACS] | = 40 mM |
| Menge(GAE) | = 500 U |
| [Mercaptoethanol] | = 1 µl/ml |

**Tabelle zu Beispiel 2:**

| Reaktionszeit bis zu quantitativem Umsatz als Funktion der Batch-Nummer *ohne* bzw. *mit* Mercaptoethanol | | |
|---|---|---|
| | *ohne* Mercaptoethanol | *mit* Mercaptoethanol [1 µl/ml] |
| X-Koeffizient [min/Batch] | 0,44 | 0,08 |
| Anzahl der Batches bis zur Verdopplung der Reaktionszeit (bei linearer Desaktivierung) [-] | 41 | 225 |

Vergleich des X-Koeffizienten (d.i. die Zeitzunahme pro Batch, also die Steigung der Geraden) zum Erreichen quantitaiven Umsatzes *ohne* bzw. *mit Mercaptoethanol*

**Tabelle 1:**

| Aminosäureanalyseprotokoll der *GAE* | | |
|---|---|---|
| Summe für die Gesamtsequenz: | | |
| Molekulargewicht = 79685.47 Anzahl der Reste = 720 Durchschnittliches Molekulargewicht pro Rest= 110.674 Ladung = -18 Isoelektrischer Punkt = 5.20 Extinktionskoeffizient = 132000 | | |
| Rest | Anzahl | Molprozent .. |
| A = Ala | 85 | 11.806 |
| B = Asx | 0 | 0.000 |
| C = Cys | 0 | 0.000 |
| D = Asp | 49 | 6.806 |
| E = Glu | 32 | 4.444 |
| F = Phe | 31 | 4.306 |
| G = Gly | 55 | 7.639 |
| H = His | 13 | 1.806 |
| I = Ile | 23 | 3.194 |
| K = Lys | 11 | 1.528 |
| L = Leu | 57 | 7.917 |
| M = Met | 14 | 1.944 |
| N = Asn | 30 | 4.167 |
| P = Pro | 55 | 7.639 |
| Q = Gln | 34 | 4.722 |
| R = Arg | 52 | 7.222 |
| S = Ser | 37 | 5.139 |
| T = Thr | 45 | 6.250 |
| V = Val | 49 | 6.806 |
| W = Trp | 16 | 2.222 |
| Y = Tyr | 32 | 4.444 |
| Z = Glx | 0 | 0.000 |
| | | |
| A + G | 140 | 19.444 |
| S + T | 82 | 11.389 |
| D + E | 81 | 11.250 |
| D + E + N + Q | 145 | 20.139 |
| H + K + R | 76 | 10.556 |
| D + E + H + K + R | 157 | 21.806 |
| I + L + M + V | 143 | 19.861 |
| F + W + Y | 79 | 10.972 |

## Patentansprüche

1. Verfahren zur Verzögerung der Desaktivierung von Glutarylamidase während einer Enzymkatalyse, **dadurch gekennzeichnet, dass** das Enzym mit mindestens einem Thiol in Kontakt gebracht wird.

2. Verfahren nach Anspruch 1, **dadurch gekennzeichnet, dass** die Glutarylamidase an einen polymeren Träger gekoppelt ist.

3. Verfahren nach Anspruch 2, **dadurch gekennzeichnet, dass** als polymerer Träger ein oxiranaktiviertes Polyacrylat verwendet wird.

4. Verfahren nach einem oder mehreren der Ansprüche nach 1 bis 3, **dadurch gekennzeichnet, dass** als Thiol 2-Mercaptoethanol verwendet wird.

5. Verfahren nach Anspruch 4, **dadurch gekennzeichnet, dass** die Konzentration von 2-Mercatoethanol im Reaktionsansatz im Bereich 1 bis 100 mM liegt.

6. Verfahren nach einem oder mehreren der Ansprüche nach 1 bis 3, **dadurch gekennzeichnet, dass** als Thiol Cystein verwendet wird.

7. Verfahren nach Anspruch 6, **dadurch gekennzeichnet, dass** die Konzentration von Cystein im Bereich 1 bis 100 mM liegt.

8. Verfahren nach einem oder mehreren der Ansprüche 1 bis 7, **dadurch gekennzeichnet, dass** bei der Enzymkatalyse mindestens ein Thiol kontinuierlich in der Substratlösung vorliegt.

9. Verfahren nach einem oder mehreren der Ansprüche 1 bis 7, **dadurch gekennzeichnet, dass** das Enzym nach dessen Abtrennung von der Substratlösung mit mindestens einem Thiol in Kontakt gebracht wird.

10. Verfahren nach einem oder mehreren der Ansprüche 1 bis 9, **dadurch gekennzeichnet, dass** Glutaryl-7-Aminocephalosporinsäure als Substrat der Enzymkatalyse verwendet wird.

11. Verfahren nach Anspruch 10, **dadurch gekennzeichnet, dass** Glutaryl-7-Aminocephalosporinsäure im Konzentrationsbereich 5 bis 500 mM verwendet wird.

12. Verfahren zur Herstellung von 7-Aminocephalosporinsäure unter Verwendung von Glutarylamidase als Enzymkatalysator, **dadurch gekennzeichnet, dass** die Desaktivierung der Glutarylamidase mittels eines Verfahrens gemäß einem oder mehreren der Ansprüche 1 bis 11 verzögert wird.

13. Verwendung von mindestens einem Thiol in einem Verfahren gemäß einem oder mehreren der Ansprüche 1 bis 12 zur Verzögerung der Desaktivierung von Glutarylamidase.

## Claims

1. A process for delaying the deactivation of glutaryl amidase during enzyme catalysis, which comprises bringing the enzyme into contact with at least one thiol.

2. The process as claimed in claim 1, wherein the glutaryl amidase is coupled to a polymeric support.

3. The process as claimed in claim 2, wherein the polymeric support used is an oxirane-activated polyacrylate.

4. The process as claimed in one or more of claims 1 to 3, wherein the thiol used is 2-mercaptoethanol.

5. The process as claimed in claim 4, wherein the concentration of 2-mercaptoethanol in the reaction mixture is in the range from 1 to 100 mM.

6. The process as claimed in one or more of claims 1 to 3, wherein the thiol used is cysteine.

7. The process as claimed in claim 6, wherein the concentration of cysteine is in the range from 1 to 100 mM.

8. The process as claimed in one or more of claims 1 to 7, wherein, during the enzyme catalysis, at least one thiol is continuously present in the substrate solution.

9. The process as claimed in one or more of claims 1 to 7, wherein the enzyme is, after its removal from the substrate solution, brought into contact with at least one thiol.

10. The process as claimed in one or more of claims 1 to 9, wherein the substrate of the enzyme catalysis used is glutaryl-7-aminocephalosporic acid.

11. The process as claimed in claim 10, wherein glutaryl-7-aminocephalosporic acid is used in the concentration range from 5 to 500 mM.

12. A process for preparing 7-aminocephalosporic acid using glutaryl amidase as enzyme catalyst, which comprises delaying the deactivation of glutaryl amidase by a process as claimed in one or more of claims 1 to 11.

13. The use of at least one thiol in a process as claimed in one or more of claims 1 to 12 for delaying the deactivation of glutaryl amidase.

## Revendications

1. Procédé pour retarder la désactivation de la glutarylamidase pendant une catalyse enzymatique **caractérisé en ce que** l'enzyme est mise en contact avec au moins un thiol.

2. Procédé selon la revendication 1, **caractérisé en ce que** la glutarylamidase est couplée à un support polymère.

3. Procédé selon la revendication 2, **caractérisé en ce qu'**un polyacrylate activé par l'oxirane est utilisé comme support polymère.

4. Procédé selon une ou plusieurs des revendications 1 à 3, **caractérisé en ce que** le 2-mercaptoéthanol est utilisé comme thiol.

5. Procédé selon la revendication 4, **caractérisé en ce que** la concentration du 2-mercaptoéthanol dans le mélange réactionnel est située dans le domaine de 1 à 100 mM.

6. Procédé selon une ou plusieurs des revendications 1 à 3, **caractérisé en ce que** la cystéine est utilisée comme thiol.

7. Procédé selon la revendication 6, **caractérisé en ce que** la concentration de la cystéine est située dans le domaine de 1 à 100 mM.

8. Procédé selon une ou plusieurs des revendications 1 à 7, **caractérisé en ce qu'**au moins un thiol est présent en continu dans la solution de substrat lors de la catalyse enzymatique.

9. Procédé selon une ou plusieurs des revendications 1 à 7, **caractérisé en ce que** l'enzyme est mise en contact avec au moins un thiol après sa séparation d'avec la solution de substrat.

10. Procédé selon une ou plusieurs des revendications 1 à 9, **caractérisé en ce que** l'acide glutaryl-7-aminocéphalosporanique est utilisé comme substrat de la catalyse enzymatique.

11. Procédé selon la revendication 10, **caractérisé en ce que** l'acide glutaryl-7-aminocéphalosporanique est utilisé dans le domaine de concentration de 5 à 500 mM.

12. Procédé de production d'acide 7-aminocéphalosporanique avec la glutarylamidase comme catalyseur enzymatique, **caractérisé en ce que** la désactivation de la glutarylamidase est retardée au moyen d'un procédé selon une ou plusieurs des revendications 1 à 11.

13. Utilisation d'au moins un thiol dans un procédé selon une ou plusieurs des revendications 1 à 12 pour retarder la désactivation de la glutarylamidase.
